(19) Europäisches Patentamt
European Patent Office
Office européen des brevets

(11) Publication number: **0 097 994**
**B1**

(12) # EUROPEAN PATENT SPECIFICATION

(45) Date of publication of patent specification: **30.09.87**

(51) Int. Cl.$^4$: **C 07 K 1/04**

(21) Application number: **83200889.0**

(22) Date of filing: **17.06.83**

(54) **Method for the solid-phase synthesis of retro-inverso polypeptides.**

(30) Priority: **24.06.82 IT 2204682**

(43) Date of publication of application:
**11.01.84 Bulletin 84/02**

(45) Publication of the grant of the patent:
**30.09.87 Bulletin 87/40**

(84) Designated Contracting States:
**AT BE CH DE FR GB LI LU NL SE**

(56) References cited:
**US-A-3 862 114**

**CHEMICAL ABSTRACTS, vol 99, 1983, page 690, no. 122860v, Columbus, Ohio, US; A. PESSI et al.: "Solid phase synthesis of retro-inverso peptide analogs. Synthesis of a partially modified retro-inverso analog of substance P, Glp6, gPhe8, mGly9rSP6-11 gPhe = -NHCH(CH2Ph)NH-, mGly = -OCCH2CO-r", & J. CHEM. SOC., CHEM. COMMUN. 1983, (4), 195-7**

(73) Proprietor: **ENICHEM S.p.A.
Via Mozart 1
I-20122 Milano (IT)**

(72) Inventor: **Pessi, Antonello
Via Massaciuccoli 19
I-00199 Rome (IT)**
Inventor: **Pinori, Massimo
Via Tevere 18
I-00015 Monterotondo (Rome) (IT)**
Inventor: **Verdini, Antonio Silvio
Via S. Martino 12
I-00015 Monterotondo (Rome) (IT)**
Inventor: **Viscomi, Giuseppe Claudio
Via 8 Maggio 23
I-00015 Monterotondo (Rome) (IT)**

(74) Representative: **Roggero, Sergio et al
Ing. Barzanò & Zanardo Milano S.p.A. Via Borgonuovo 10
I-20121 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 097 994 B1**

(56) References cited:

**CHEMICAL ABSTRACTS, vol. 99, 1983, page 638, no. 140359t, Columbus, Ohio, US; A. PESSI : "Solid phase synthesis of retro-inverso peptides. Synthesis of the partially modified retro-inverso analog of substance P Glp6, gPhe8, mGly9rSP6-11", & FARMACO, ED. SCI. 1983, 38(5), 360-8**

**CHEMICAL ABSTRACTS, vol. 94, 1981, page 693, no. 157231q, Columbus, Ohio, US; M. CHOREV et al.: "Novel partially modified retro-inverso analogs of biologically active peptides.", & PEPT., STRUCT. BIOL. FUNCT., PROC. AM. PEPT. SYMP., 6th 1979, 455-8**

**Description**

Inversion of the direction of the amide bonds of peptide chains

(from ∿ NH—CH—CO→NH— CH—CO ∿ to ∿ NH—CH—NH←CO— CH—CO ∿)
R R' R R'

enables biologically active peptide analogues to be obtained which besides being an interesting object of study in that they make it possible to determine the relative importance of the side chains and peptide skeleton in biological activity, can find useful practical application in pharmacology because of their high stability to enzymatic degradation (Goodman M and Chorev M, Acc. Chem. Res. *12,* 1, (1979) and cited references). The resultant analogues known generally as retro-inverso peptides, are structural isomers which although not exactly identical with the natural reference peptides from the topochemical aspect, can retain their biological activity. In particular, in the case of cyclic peptides and depsipeptides it has been shown that inversion of the direction of the peptide skeleton accompanied by inversion of each of the chiral centres of the chain produces structural isomers with complete biological activity (Shemyakin M.M., Ovchinnikov Y.A. and Ivanov V.T, Angew. Chem. 8, 492 (1969)). It is apparent that in these compounds the biological activity depends on the three-dimensional orientation of the side chains rather than on the structure of the peptide skeleton.

In the case of retro-inverso analogues of linear peptides with all the peptide bonds inverted, the existence of the terminal groups poses particular problems for maintaining the steric complementarity with the reference peptide.

In general, and in the case of linear reference peptides with amino and carboxyl free chain terminals, the problem is solved by transforming the N-terminal amino acid residue into a gem-diamino residue, transforming the C-terminal amino acid residue into a malonyl residue or a malonyl residue substituted in position 2, and inserting the intermediate residues into the chain with inverted configuration. If not all the peptide bonds of the chain are retro-inverted, partially modified retro-inverso analogues are obtained in which the modified chain segments are incorporated between normal segments. The inversion of a single peptide bond produces two adjacent non-amino acid residues in which the residue closer to the amino end is of gem-diamino type, whereas that closer to the carbonyl end is of malonyl or 2-substituted malonyl type.

The extension of the modification to non-adjacent peptide bonds leads to inversion of the chirality of the amino acids interposed between the two non-amino acid residues. Up to the present time, biologically active retro-inverso analogues of linear peptides have been synthesised such as angiotensin, bradykinin, luteotropic hormone releasing hormone, thyrotropin releasing hormone, enkephalin, tuftsin, C-terminal tetrapeptide of desamino gastrin, 5—9 pentapeptide of α-melanotropin, substance $P_{5-11}$, aspartame and enkephalinase inhibitors.

(Goissis G. et al., J. Med. Chem. 19, 1287 (1976); Vogler K. et al., Helv. Chim. Acta, 49, 390 (1966); Chorev M. et al., in "Peptides: Proceedings of the 5th American Peptide Symposium", Goodman M. and Meienhofer J., Editors, Wiley, New York 1977 p. 572; Goodman M. and Chorev M. in "Perspectives in Peptide Chemistry", Eberle A., Geiger R. and Vieland T., Editors, Karger, Basel 1980 p. 283; Chorev M. et al., Science 204, 1210 (1979); Hayward C. F. and Morley J. S. in "Peptides 1974, Proceedings of the 13th European Peptide Symposium", Volman Y. Ed., Wiley, New York and Israel Universities Press, Jerusalem, 1975, p. 287, Chung D. and Li C.H., Biochim. Biophys. Acta 136, 570 (1967); Cuignet E. et al. in "Peptides 1980, Proceedings of the 16th European Peptide Symposium", Brunfeldt K. Ed., Scriptor, Copenhagen, 1981 p. 608; M.C. Fournie — Zaluski et al. in "Proceedings of the 7th American Peptide Symposium", D.H. Rich and E. Gross Editori, Pierce Chemical Co., Rockford, 1982, p. 425) in particular, the synthesis of enkephalinamide analogues with inversion of the peptide bond between residues 4 and 5, with or without simultaneous inversion of the terminal carboxyamide bond, has produced new and powerful topochemical analogues of the enkephalins.

In this case, the inversion of part of the amide bonds of the chain has enabled not only the biological activity to be preserved unaltered but also to potentiate it, probably by the effect of the increased resistance to enzymatic hydrolysis in vivo.

While the incorporation of the malonyl or 2-substituted malonyl residues into the skeleton of these peptides has not presented particular problems, that of the gem-diaminoalkyl residues has required special and delicate synthetic manipulations. The sequence of reactions used can be summarised as follows: an amino acid or peptide-azide suitably protected at the $NH_2$ is firstly converted into the corresponding isocyanate by means of Curtis rearrangement. The isocyanate intermediate is then subsequently utilised either in reactions with the carboxyl component to give the mixed carbamic-carboxylic anhydride, which by thermal elimination of carbon dioxide produces the desired amide bond, or is captured by reaction with an excess of an appropriate alcohol (in general tertiary butyl or benzyl alcohol) to give a new type of urethan protecting group for the gem-diamino residue.

This new amino protecting group is eliminated immediately before the condensation reaction with the carboxyl component, because of the instability of the gem-diaminoalkyl derivatives (Goodman M and

Chorev M., Acc. Chem. Res. 12, 1, (1979)).

The isocyanate intermediates are prepared in their turn by three different synthetic procedures:

a) reacting the carboxyl component with diphenylphosphorylazide in toluene, followed by heating in the presence of triethylamine (Wilson C.G. et al. in "Peptides: Proceedings of the 5th American Peptide Symposium", Goodman M. and Meienhofer J., Editors, Wiley, New York 1977, p. 579);

b) reacting the mixed carbamic-carboxylic anhydride under cold conditions with an excess of sodium azide in water to give the corresponding acyl azide, followed by thermal rearrangement (Sheehan J.C. et al., J. Org. Chem. 42, 4045 (1977));

c) reacting an acyl hydrazide with nitrosyl chloride in anhydrous tetrahydrofuran to give the corresponding azide, followed by thermal rearrangement (Honzl-Rudinger process) (Honzl J. and Rudinger J., Coll. Czech. Chem. Commun. 26, 2333 (1961)).

We have recently devised a method which makes it possible to introduce a gem-diamino residue into a peptide skeleton in a particularly easy manner without special tedious chemical manipulation, by using I,I-bis(trifluoroacetoxy)iodobenzene. This reagent has been previously used for the direct conversion of primary carboxyl amides of simple structure into amines under extremely mild reaction conditions without the need to isolate or capture the intermediate isocyanate (Radhakrishna A.S. et al., J. Org. Chem. 44, 1746 (1979)). Our method, described in copending patent applications in the name of the same applicant, is easily applied to the direct conversion of primary amino acid or peptide amides protected at the terminal $NH_2$, into corresponding trifluoroacetic acid salts of N-monoacylated gem-diamino derivatives. We have thus been able to very easily synthesise, in the homogeneous phase, partially retro-inverso analogues of C-terminal penta and hexapeptides of Substance P, of enkephalin, of bradykinin potentiating pentapeptide (BPP 5a) and the Haber renin inhibitor (Burton et al., U.S. Patent 4,269,827). We have now found that I,I-bis(trifluoroacetoxy)iodobenzene can be advantageously used under mild reaction conditions for *converting, into amines, primary amides of polypeptide chains synthesised on a polymer matrix and still covalently bonded to the solid support,* to thus obtain the same advantages as in the solid-phase synthesis of polypeptides, ie:

a) the peptide is synthesised while covalently bonded to an insoluble support, this allowing easy elimination of the soluble reaction by-products;

b) the reactions at the peptide chains bonded to the polymer matrix can be carried out exhaustively using suitable excesses of soluble reagents, which are eliminated when required by filtration and thorough washing;

c) losses deriving from the manipulations necessary for isolating and purifying the products are prevented because during the course of the synthesis the solid support with the growing peptide is kept in a single container;

d) the manipulations are easy, rapid and can be automated. (G. Barany and R. B. Merrifield, "The Peptides" vol. 2, E. Gross and J. Meienhofer Editors, Academic Press, New York, 1980 p.1). It follows that the combined use of the advantages offered both by the solid-phase synthesis of primary polypeptide amides and by their direct conversion into the corresponding amines on the same polymer support matrix enables biologically active polypeptide analogues to be obtained with one or more retro-inversions of the peptide bonds in different chain positions, by means of synthetic strategies and procedures which are not very dissimilar to those already applied to the solid-phase assembly of the natural polypeptide amino acid sequences, and still compatible with automation of the method. Of the resins used in solid-phase polypeptide synthesis (copolystyrene-divinylbenzene, copolyacrylamide-N,N'-methylenebisacrylamide, polydextran hydroxypropylate, polystyrene on glass, polystyrene on polystyrene, polystyrene on Kel-F, cross-linked polyethylene amine absorbed on macroporous alumina, polydimethylacrylamide-co-Boc-p-Ala-N'-acryloylhexamethylenediamine cross-linked with N,N'-ethylene-bis-acrylamide, polydimethylacrylamide-co-acryloylsarcosinemethylester cross-linked with N,N'-ethylene-bisacrylamide, and poly(N-acryloylpyrrolidine)-co-N-acryloylhexamethylenediamine cross-linked with N,N'-ethylene-bis-acrylamide), the last three are mostly compatible with the aqueous-organic solvent mixtures necessary for using I,I-bis(trifluoroacetoxy)iodobenzene. We have now discovered that which constitutes the subject matter of the present invention, namely that it is possible to synthesise, *entirely in the solid phase,* polypeptides with one or more retroinverted peptide bonds by using:

a) polydimethylacrylamido-co-acryloylsarcosinemethylester resin cross-linked with N,N'-ethylene-acrylamide (Sheppard's resin);

b) the reagent I,I-bis(trifluoroacetoxy)iodobenzene; and

c) a synthetic strategy comprising assembling the polypeptide chains starting from the C-terminal residue by successive condensation of suitable derivatives of individual amino acids and compounds corresponding to general formula

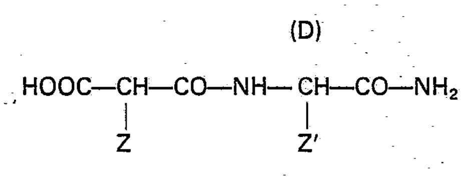

(I)

where Z and Z′, which can be the same or different, indicate the side-chain groups of the amino acids commonly present in the natural polypeptide molecules, with final simultaneous release of the entire peptide from the resin, of the terminal $NH_2$ protector groups, and of the functionalities of the amino acid side-chains compatible with the release conditions used.

The new synthesis method described in the present patent can be schematised as follows.

| | |
|---|---|
| Ⓡ ↓ | introduction of function group (x) |
| X—Ⓡ ↓ | condensation of the internal standard (s), protected by a temporary protector group (B) |
| B—S—X—Ⓡ ↓ | condensation of the peptide-resin "handle" (H) |
| H—S—X—Ⓡ ↓ | condensation of the C-terminal amino acid residue ($A_1$) |
| B—$A_1$—H—S—X—Ⓡ ↓ | peptide chain elongation (A—. . .) |
| B—A—. . .$A_1$—H—S—X—Ⓡ ↓ | condensation of derivatives of general formula I (A—M) |
| A—M—A—. . .—$A_1$—H—S—X—Ⓡ ↓ | amide-amine conversion ($A_m$—M) and retro-inversion of peptide bond |
| $A_m$—H—A . . . .—$A_1$—H—S—X—Ⓡ ↓ | peptide chain elongation |
| . . .B—A—. . .—Am—M—A—. . .—$A_1$—H—S—X—Ⓡ ↓ | detachment at the resin and deprotection according to the synthesis objective |
| B—A—. . .—Am—M—A—. . .—$A_1$ a) | |
| B—A—. . .—Am—M—A—. . .—$A_{—1}$—P b) | |
| A—. . .—Am—M—A—. . .—$A_1$—P c) ↓ | total deprotection |
| A—. . . .—Am—M—A—. . .—$A_1$ | |

where $X = NH_2$; S = norleucine residue; H = peptide-resin connection hook; $A_1$ = C-terminal amino acid residue; A = amino acid residue; A—M = malonyl or 2-substituted malonyl derivatives of primary amides of D-amino acids; $A_m$ = gem-diamino or 2-substituted gem-diamino residue; P = peptide terminal carboxyl protector group; a, b, c = selective release conditions to obtain, respectively, peptides with the amino end protected, peptides with the amino and carboxyl end protected, and peptides with the carboxyl end protected. This method of synthesis also allows isolation of peptide fragments selectively protected at the $—NH_2$ and/or —COOH end and/or at the side-chains of the amino acids, for their further use (fragment coupling) in the synthesis of polypeptides of more complex structure. We have chosen to use N-9-fluorenyl-methoxycarbonylamino acids (Fmoc-amino acids) in combination with amino acid side-chain protecting groups of t-butyl and/or p-alkoxybenzyl type and with the peptide-resin bond of p-alkoxybenzyl type (E. Atherton et al., J. Chem. Soc. Perkin I. 538 (1981)).

The choice of these protecting groups enables the solid-phase synthesis of retro-inverso peptides to be conducted under exceptionally mild reaction conditions, and in particular without compromising the integrity of the gem-diamino residues inserted into the polypeptide skeleton. The resin used for the synthesis of the present invention can be of commercial origin or can be prepared by copolymerisation in an emulsion of dimethylacrylamide, acryloylsarcosine methyl ester and bis-acryloylethylenediamine, initiated by ammonium persulphate in accordance with the procedure described in the aforesaid publication of Atherton and colleagues. The amino acid derivatives most frequently used in the synthesis of the retroinverso peptides according to the present invention are listed in Table 1.

TABLE 1

| Amino acid derivatives | Symbol |
|---|---|
| $N^\alpha$-9-fluorenylmethoxycarbonyl-alanine | Fmoc-Ala |
| $N^\alpha$-9-fluorenylmethoxycarbonyl-leucine | Fmoc-Leu |
| $N^\alpha$-9-fluorenylmethoxycarbonyl-glycine | Fmoc-Gly |
| $N^\alpha$-9-fluorenylmethoxycarbonyl-valine | Fmoc-Val |
| $N^\alpha$-9-fluorenylmethoxycarbonyl-proline | Fmoc-Pro |
| $N^\alpha$-9-fluorenylmethoxycarbonyl-isoleucine | Fmoc-Ile |
| $N^\alpha$-9-fluorenylmethoxycarbonyl-methionine | Fmoc-Met |
| $N^\alpha$-9-fluorenylmethoxycarbonyl-phenylalanine | Fmoc-Phe |
| $N^\alpha$-9-fluorenylmethoxycarbonyl-tryptophan | Fmoc-Trp |
| $N^\alpha$-9-fluorenylmethoxycarbonyl-asparagine | Fmoc-Asn |
| $N^\alpha$-9-fluorenylmethoxycarbonyl-glutamine | Fmoc-Gln |
| Butyl $N^\alpha$-9-fluorenylmethoxycarbonyl-γ-glutamate | Fmoc (γ-tBu)-Glu |
| t-butyl $N^\alpha$-9-fluorenylmethoxycarbonyl-β-aspartate | Fmoc (β-tBu)-Asp |
| $N^\alpha$-9-fluorenylmethoxycarbonyl-$N^\varepsilon$-trifluoroacetyl-lysine | Fmoc ($N^\varepsilon$—TFA)-Lys |
| $N^\alpha$-9-fluorenylmethoxycarbonyl-$N^\varepsilon$-t-butoxy-carbonyl-lysine | Fmoc ($N^\varepsilon$—BOC)-Lys |
| $N^\alpha$-9-fluorenylmethoxycarbonyl-O-t-butyl-serine | Fmoc (O-tBu)-Ser |
| $N^\alpha$-9-fluorenylmethoxycarbonyl-O-t-butyl-threonine | Fmoc (O-tBu)-Thr |
| $N^\alpha$-9-fluorenylmethoxycarbonyl-$N^J$,$N^\omega$-bis-adamantyloxycarbonyl-arginine | Fmoc$N^J N^\omega(Adoc)_2$-Arg |

TABLE 1 (continued)

| Amino acid derivatives | Symbol |
|---|---|
| $N^{\alpha}$-9-fluorenylmethoxycarbonyl-$N^{\omega}$-p-methoxy-benzenesulphonylarginine | Fmoc ($N^{\omega}$—Mbs)-Arg |
| $N^{\alpha}$-9-fluorenylmethoxycarbonyl-arginine hydrochloride | Fmoc-Arg.HCl |
| $N^{\alpha}$-9-fluorenylmethoxycarbonyl-O-t-butyl-tyrosine | Fmoc (O-tBu)-Tyr |
| $N^{\alpha}$-9-fluorenylmethoxycarbonyl-S-t-butyl-cysteine | Fmoc (S-tBu)-Cys |
| $N^{\alpha}$-9-fluorenylmethoxycarbonyl-S-acetamido-methyl-cysteine | Fmoc (S—Acm)-Cys |
| $N^{\alpha}$-9-fluorenylmethoxycarbonyl-$N^{im}$-9-fluorenylmethoxycarbonylhistidine | Fmoc ($N^{im}$—FMOC)-His |
| $N^{\alpha}$-t-butyloxycarbonyl-$N^{im}$-t-butyloxy-carbonyl-histidine | Boc ($N^{im}$—Boc)-His |
| $N^{\alpha}$-t-butyloxycarbonyl-$N^{\pi}$-benzyloxy-methyl-histidine | Boc ($N^{\pi}$—BOM)-His |
| $N^{\alpha}$-9-fluorenylmethoxycarbonyl-$N^{\pi}$-benzyloxymethyl-histidine | Fmoc ($N^{\pi}$—BOM)-His |

Table 2 shows the peptide-resin "handle" and the conditions for their release.

TABLE 2

| "Handle" | Resin release conditions |
|---|---|
| 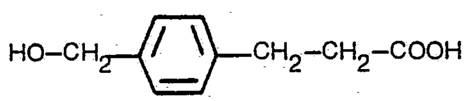 | Liq. HF; ammonolysis ($NH_3$ in $CH_3OH$) |
| 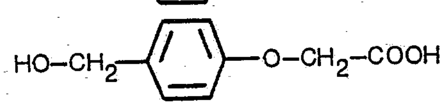 | Acidolysis |
| 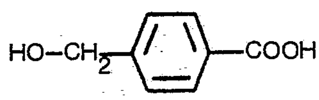 | Catalytic hydrogenolysis; ammonolysis |
| 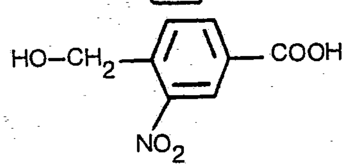 | Photolysis |
| 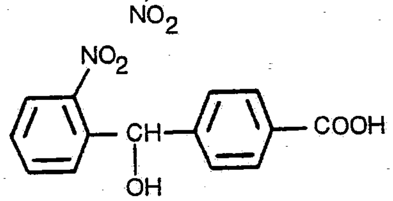 | Photolysis |
| 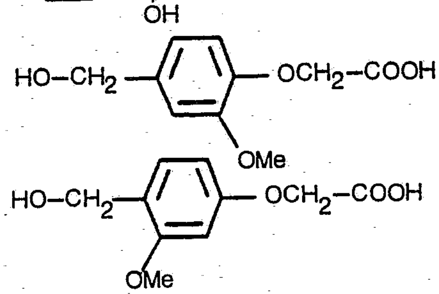 | Acidolysis (dilute acids) |
|  | Acidolysis (dilute acids) |

The resin functionalisation reaction with primary amino groups is effected by ethylenediamine, and the condensation of the reference standard and spacer is effected using the symmetrical anhydride of Fmoc-norleucine, while the peptide-resin "handle" are bonded to the modified polymer by using symmetrical or their anhydrides 2,4,5-trichlorophenyl esters. To the resins functionalised in this manner, and which respond to the following general formulas:

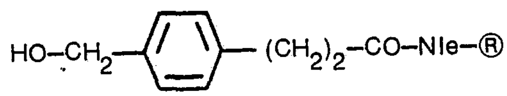

(II)

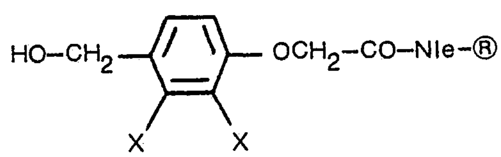

(III)

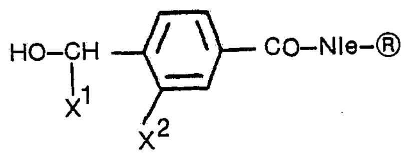

(IV)

where X = H or —OMe, $X^1$ = H or

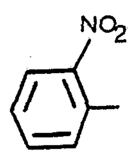
and $X^2 = NO_2$,

there is covalently bonded under the conditions described by Atherton and colleagues (E. Atherton et al., J. Chem. Soc., Chem. Com. 336, (1981)), the C-terminal amino acid residue with its $NH_2^\alpha$ protected by the Fmoc group, whereas the subsequent amino acid residues are introduced, in the form of $NH_2^\alpha$-Fmoc derivatives, by the standard procedure of Table 3.

TABLE 3

| $^{\alpha}$-Fmoc-$^{\omega}$-butyl | | Time (min.) |
|---|---|---|
| | Wash with DFM(*)or | |
| | DMA | 1 × 5 |
| Deprotection | Piperidine (PIPD)—DMF | 1 × 3; 1 × 7 |
| | Wash with DMF | 1 × 10 |
| Condensation | Fmoc/BOC-aminoacid-anhydride or active ester/N-hydroxy-benzotriazole-DMF | 60—120 |
| | Wash with DMF | 1 × 5 |

(*)The solvent used during the entire synthesis is dimethylformamide, which hereinafter will be indicated by the symbol DMF, or dimethylacetamide (DMA).

The condensation of the products corresponding to general formula (I) is effected using N,N'-dicyclohexylcarbodiimide in the presence of N-hydroxybenzotriazole, or of the preformed symmetrical anhydride. In their turn, the compounds corresponding to general formula (I) can be obtained from a D amino acid primary amide by condensing with a monoester of malonic acid, or of malonic acid substituted in position 2, of general formula:

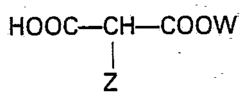

(V)

where Z has the meaning given heretofore, and W is methyl, ethyl, benzyl, or t-butyl, in the presence of a suitable condensing agent, preferably N,N'-dicyclohexylcarbodiimide, and N-hydroxybenzotriazole as additive.

The ester protecting group can be removed by catalytic hydrogenation (benzyl ester), by acidolysis (t-butyl ester) or by alkaline hydrolysis (methyl, ethyl and benzyl ester), to obtain the compounds of general formula (I).

The compounds of general formula (I), where Z is H or $CH_3$, can be also more rapidly synthesised by treating D amino acid primary amides with Meldrum acid or its derivative (P. Crooy et al., Bull. Soc. Chim. Belg. 86, 991 (1977) and cited references), in accordance with the reaction scheme:

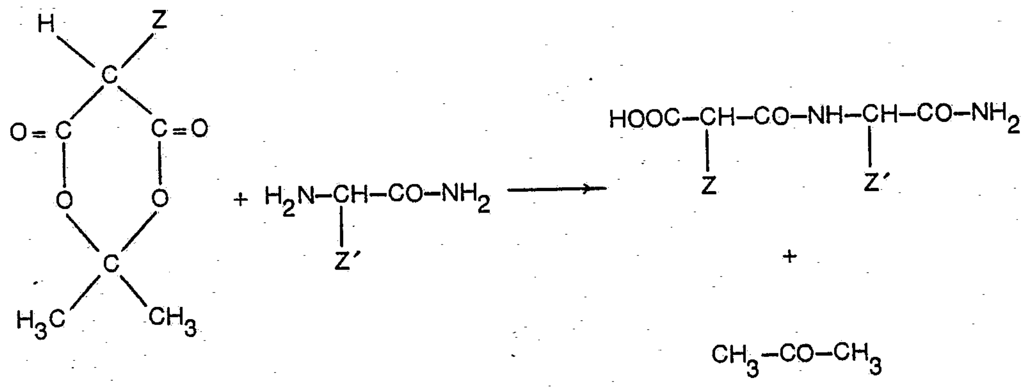

where Z and Z' have the aforesaid meanings.

The conversion, into amines of the terminal carboxyamide group on the resins swollen in $H_2O$—DMF is effected by treatment with I,I-bis-(trifluoroacetoxy)iodobenzene (BTI) dissolved in DMF, followed by careful washing with $H_2O$—DMF and DMF in order to remove all traces of residual $H_2O$ on the resin, which could compromise the subsequent condensation reactions. After neutralising the trifluoroacetate salt by washing with ethyldiisopropylamine followed by washing with DMF, the peptide chain is elongated by treatment with suitably protected reactive amino acid derivatives, by the typical solid-phase synthesis method.

The degree of completion of the acylation reaction is evaluated by the qualitative ninhydrin test of Kaiser and colleagues (E. Kaiser et al., Analyt. Biochem. 34, 595 (1970)) and by the 2,4,6-trinitrobenzenesulphonic acid test (TNBS) (J.K. Immam and H.M. Dintzis, Biochemistry, 8, 4074 (1969)). After detaching the peptides from the resin by the standard acidolytic, hydrogenolytic or photolytic reactions, or by ammonolysis in the presence of methanol, the pure products are obtained by known peptide isolation processes such as extraction, counter-current distribution, precipitation, crystallisation and various chromatographic methods.

The identity of the retro-inverso peptides is established by the combined use of protonic nuclear magnetic resonance, infrared absorption, mass spectrometry, enzymatic hydrolysis, amino acid analysis and amino acid sequence determination. The subject matter and the object of the invention will be more apparent from the example given hereinafter for illustrative purposes only, and which must in no way be considered as limitative of the invention.

## Example

Synthesis of pyroglutamylphenylalanyl-gem-diaminophenylalanylmalonylleucylmethionineamide. Glp-Phe-gPhe-mGly-Leu-Met-$NH_2$.

1) Synthesis of the resin Copoli (dimethylacrylamide-bis-acryloylethylenediamine-acryloylsarcosinemethylester).

The copolymer was synthesised as described by Arshady and colleagues (R. Arshady et al., J. Chem. Soc., Perkin I, 529 (1981)). 12.5 g of cellulose acetate butyrate are dissolved in 300 ml of $CH_2Cl_2$ and placed in a cylindrical polymerisation vessel comprising an agitator and an inert gas inlet, and kept at 50 ± 1°C in a temperature-controlled bath. The solution is agitated at 450 ± 20 r.p.m. and flushed with $N_2$ before adding a mixture consisting of 15 g (1.52 mmoles) of dimethylacrylamide, 1.25 g (7.96 mmoles) of acryloylsarcosinemethylester, 1.75 g (10.4 mmoles) of bis-acryloylethylenediamine, diluted with 150 ml of cold (approx. 5°C) DMF—$H_2O$ (1:2 v/v) and well mixed with 2.25 g of ammonium persulphate. Polymerisation is carried out for about 15 hours under a very slight flow of $N_2$. The mixture is cooled, diluted with $(CH_3)_2CO$—$H_2O$ (1:1 v/v), agitated until a homogeneous suspension is obtained, and then filtered.

The recovered polymer is washed thoroughly, and the pulverulent particles are removed by agitation and by decanting with $(CH_3)_2CO$—$H_2O$ (1:2 v/v) (3 × 1 litre) and $(CH_3)_2Co$ (3—4 × 500 ml). The polymer is finally washed with ether (2 × 500 ml), collected by filtration and dried over $P_2O_5$ under vacuum. Yield 15 g

of resin in the form of spherical particles (found: Sar, 1.04 milliequiv/g.).

2) Functionalisation of the resin with ethylenediamine.

The resin is functionalised directly in the reaction vessel immediately before the synthesis.

0.5 g of resin are suspended in 16 ml of distilled ethylenediamine and shaken overnight at ambient temperature.

The reaction vessel is drained, and the resin washed with DMF (10 × 1 min), DIPEA (10%) in DMF (3 × 2 min) and then DMF (5 × 2 min).

3) Condensation of the internal norleucine standard at the $NH_2$ groups of the resin functionalised with ethylenediamine

0.9 mmoles of $(Fmoc\text{-}Nle)_2O$ dissolved in 8 ml of DMF are fed into the reactor and allowed to react for 60 minutes under agitation. After draining the reaction liquid, the resin is washed with DMF (5 × 1 min). Tests with ninhydrin and TNBS show that the reaction is complete. The incorporation of Fmoc-Nle in the resin is 0.525 mmoles/g.

4) Introduction of the p-hydroxymethylbenzoic acid "handle" to Fmoc-Nle-Ⓡ.

The resin, prepared by the procedure described under (3) is treated with PIPD—DMF (20:80 v/v) (1 × 3 min), (1 × 7 min), washed with DMF (10 × 1 min) and treated with 0.9 mmoles of p-hydroxymethylbenzoic acid trichlorophenylester dissolved in 8 ml of DMF, together with 0.9 mmoles of HOBt (N-hydroxybenzotriazole). Shaking is continued overnight. When the ninhydrin and TNBS tests show that the condensation reaction is complete, the resin is washed with DMF (10 × 1 min), 10% DIPEA in DMF (3 × 1 min) and finally with DMF (10 × 1 min).

5) Condensation of the C-terminal methionine residue on the resin.

The condensation of the C-terminal residue on the resin prepared as described under point (4) is effected under the conditions described by Atherton and colleagues (E. Atherton et al., J. Chem. Soc., Chem. Comm., 336 (1981)).

0.9 mmoles of $(Fmoc\text{-}Met)_2O$ are fed into the reactor containing the resin together with 0.09 mmoles of N,N'-p-dimethylaminopyridine (DMAP) and 0.8 millimols of N-methylmorpholine (NMM), and are allowed to react under agitation for 30 minutes. After draining off the reaction liquid, the normal washes are carried out with DMF (10 × 1 min).

6) Condensation of the leucine residue on the resin.

The resin prepared by the procedure described under point (5) is treated with PIPD—DMF (20:80 v/v) (1 × 3 min), (1 × 7 min), washed with DMF (10 × 1 min) and treated with 0.9 mmoles of $(Fmoc\text{-}Leu)_2O$ dissolved in DMF.

The condensation is carried out for 60 minutes under shaking, and is interrupted when the ninhydrin and TNBS tests are negative. The resin is then washed with DMF (10 × 1 min) and used partly for analysis of amino acids by the following procedure: a portion of resin (5—10 mg) swollen in DMF is transferred on to a Gooch crucible, washed with DMF, and then treated with PIPD—DMF (20:80 v/v) (1 × 3 min) (1 × 7 min) in order to release the Fmoc group. After washing with DMF, the resin is treated in succession with methanol and ethyl ether, then dried under vacuum to remove solvent traces. A dry sample is hydrolysed with 6N HCl in a closed phial for 18 hours at 115°C.

| Amino acids | Norleucine | Methionine | Leucine |
|---|---|---|---|
| Theoretical | 1 | 1 | 1 |
| Found | 1.17 | 0.79 | 1.00 |

The leucine concentration on the resin is 0.449 mmoles/g.

7) Synthesis of the methyl ester of malonyl-D-phenylalaninamide $MeO\text{-}mGLY\text{-}D\text{-}PHE\text{-}NH_2$.

2.37 g (17.5 mmoles) of HOBt dissolved in DMF are added to 1.73 g (14.6 mmoles) of malonic acid monomethylester dissolved in $CH_2Cl_2$. The resultant mixture is cooled to 0°C, and to it are added 3.32 g (16.08 mmoles) of DCC dissolved in $CH_2Cl_2$ and, after about 40 minutes, 1.2 g (7.31 mmoles) of $D\text{-}Phe\text{-}NH_2$ dissolved in $CH_2Cl_2$/DMF. The mixture is allowed to react for 1 hour at 0°C and then for a further 16 hours at ambient temperature, the reaction flask then being brought to a temperature of −20°C for 16 hours in order to precipitate the DCU (N,N'-dicyclohexylurea) which forms. After filtering the solid through a Gooch crucible, the $CH_2Cl_2$ and DMF are evaporated. The oily residue is taken up in THF (tetrahydrofuran), filtered and again evaporated to dryness.

The desired product is crystallised from ethanol (ETOH) — petroleum ether 30—50°C.

Yield = 1.8 g (71%).

The 1H n.m.r. spectrum confirms the product identity.

8) Synthesis of malonyl-D-phenylalaninamide, $HOOC—CH_2—CO—D—Phe$.

1.3 g (5.2 mmoles) of $MeO\text{-}mGly\text{-}D\text{-}Phe\text{-}NH_2$, dissolved in MeOH (methanol), are treated with 3.5 ml of 3M NaOH in MeOH (10.4 mmoles) for a time of 40 minutes. On completion of the reaction, water is added to the reaction mixture, the MeOH is completely evaporated and the aqueous solution is concentrated to a small volume.

After adding a saturated NaCl solution, the mixture is acidified with 3N HCl to pH 2.0, and extracted

EtOAc (ethyl acetate). Having found that the desired product distributes through both the phases, the aqueous phase is concentrated almost to dryness, THF is added to precipitate NaCl, the salt is filtered off, and finally the organic solution is evaporated to an oily consistency.

The product is crystallised from EtOH/petroleum ether 30—50°C. The fraction dissolved in EtOAc is dried over $MgSO_4$, filtered, evaporated to dryness and recovered after remaining at 4°C for about 16 hours. M.P. 140—142°C.

Silica gel thin layer chromatography shows the presence of a single stain, and the 1H n.m.r. spectrum confirms the molecular structure.

9. Condensation of m-Gly-D-Phe-$NH_2$ on the resin.

The resin, prepared by the procedure described under point (6), is firstly washed with DMF (10 × 1 min), then treated with PIPD—DMF (20:80 (1 × 3 min), (1 × 7 min) and washed with DMF (10 × 1 min). 0.9 mmoles of HOBt, 0.9 mmoles of m-Gly-D-Phe-$NH_2$ and 0.9 mmoles of DCC are then fed into the reactor, and left to react under agitation for about 16 hours. When the ninhydrin and TNBS tests show that the reaction is complete, the resin is washed with DMF (15 × 1 min) in order to completely remove the DCU, and a portion thereof is used for determining the amino acid concentration by the previously described method.

| Amino acids | Norleucine | Leucine | Methionine | Phenylalanine |
|---|---|---|---|---|
| Theoretical | 1 | 1 | 1 | 1 |
| Found | 1.56 | 1.00 | 0.85 | 0.96 |

Analysis (*) shows that prolonged acylation times have produced a consistent (about 20%) loss of Leu-Met dipeptide, probably because of the formation of the corresponding diketopiperazine.

10) Complete acylation of the resin.

The resin, prepared as described under point (9), is washed with DMF (5 × 1 min) and treated with 0.1 ml of $(CH_3CO)_2O$ (0.95 mmoles) dissolved in 8 ml of DMF for 60 minutes under shaking. When the ninhydrin and TNBS tests show that the reaction is complete, the resin is finally washed with DMF (10 × 1 min).

11. Direct conversion of the terminal carboxyamide group into the amino group on the resin.

The resin, prepared as described under point (10), is swollen in the solvent mixture used for the reaction with BTI by treatment with DMF—$H_2O$ (3:1 v/v) directly in the reactor. 0.9 mmoles of BTI dissolved in 8 ml of DMF are added to the resin. The mixture is shaken for 60 minutes, the excess reagent is removed by draining, and the resin is again washed with DMF—$H_2O$ (3:1 v/v) before further adding 0.9 mmoles of BTI. The mixture is shaken for a further 60 minutes, the preceding washes are repeated, and a further 0.9 mmoles of reagent are added, followed by shaking for a further 60 minutes. After further washing with the DMF—$H_2O$ mixture (3:1 v/v), all traces of $H_2O$ are removed by washing with anhydrous DMF, and the trifluoroacetic acid salt is neutralised by the following series of washes: DMF (15 × 1 min), 10% aqueous DIPEA/DMF (3 × 1 min) and finally DMF (10 × 1 min).

12) Condensation of the phenylalanine residue on the resin.

0.9 mmoles of $(Fmoc\text{-}Phe)_2O$ dissolved in 8 ml of DMF are added in the reactor to the resin prepared by the procedure of point (11). The mixture is allowed to react for 60 minutes under agitation, It is then washed with DMF (10 × 1 min) and the addition of $(Fmoc\text{-}Phe)_2O$ is repeated as heretofore, allowing the mixture to react for 55 minutes. After carrying out the colorimetric control tests, washing is effected with DMF (5 × 1 min), and a release cycle for the terminal $NH_2$ protector group is commenced by the normal procedure: treatment with PIPD—DMF (20:80), (1 × 3 min), (1 × 7 min), followed by washing with DMF (10 × 1 min).

13) Condensation of the pyroglutamic acid on the resin.

0.9 mmoles of pyroglutamic acid pentachlorophenyl ester (Glp-OPCP) together with 0.9 mmoles of HOBt, dissolved in 7.8 ml of DMF are added in the reactor to the resin prepared as described under point (12). The mixture is shaken for 60 minutes. After carrying out the ninhydrin and TNBS colorimetric tests to check the completeness of the reaction, the final washes are effected with DMF (10 × 1 min).

The resin is then transferred onto a G—3 Gooch crucible, is washed with DMF—DIPEA (90:10), DMF, MeOH and finally ethyl ether ($Et_2O$), and dried under vacuum to constant weight. Final resin weight = 0.600 g. A dry resin sample is hydrolysed with 6N HCl for 18 hours in order to determine the amino acid composition of the synthesised peptide:

| Aminoacid | Norleucine | Leucine | Methionine | Methionine* sulphoxide | Phenylalanine | Glutamic acid |
|---|---|---|---|---|---|---|
| Theoretical | 1 | 1 | 1 | — | 1 | 1 |
| Found | 1.51 | 1.00 | 0.47 | 0.62 | 0.77 | 0.61 |

Peptide loading on resin = 0.239 mmoles/g

Calculated with aspartic acid extinction coefficient in accordance with Spackman and colleagues (Spackman et al., Anal. Chem. 30, 1190, 1185 (1958)).

14) Release of the peptide from the resin by ammonolysis.

0.250 g of dry resin are left to swell completely for 30 minutes in 4 ml of DMF. After adding 50 ml of MeOH and cooling to 0°C, $NH_3$ is bubbled through until the resin suspension is saturated. The temperature is maintained at 0°C for two hours, after which it is allowed to rise to ambient temperature while continuing bubbling of $NH_3$ for a further two hours. On termination, the resin is filtered, washed thoroughly with MeOH, the various portions of solution are combined and evaporated to dryness. After adding $Et_2O$, the mixture is again evaporated to dryness, the flask finally being left under vacuum for about 150 minutes.

The residual resin is analysed to check the degree of completeness of the release by firstly carrying out a series of washes with DMF, MeOH and $Et_2O$, drying under vacuum and hydrolysing a suitable portion in 6N HCl for 18 hours. The result obtained, (Leucine)/Norleucine) = 0.028, indicates that the extent of release is 97% of the theoretical.

15) Chromatographic analysis of the crude reaction product pyroglutamylphenylalanyl-gem-diaminophenylalanylmalonyl-leucylmethionineamide.

The product, obtained as described under point (14), was analysed by reverse phase high pressure liquid chromatography, the stationary phase being constituted by Lichrosorb RP 18 (5 µm) (Merck), the eluent being $H_2O$—$CH_3CN$ (73:27), after initially dissolving in HFIP (hexafluoroisopropanol).

The reference standard used was a sample of the same retro-inverso hexapeptide already prepared in the homogeneous phase as described in a copending patent application in the name of the same applicant. (European Pat. Appln. N° 82201612.7). The main peak of the chromatogram corresponds to the peptide with the methionine residue substituted by a methionine sulphoxide residue, produced during treatment with BTI, as demonstrated by means of exhaustive oxidation experiments on the standard retro-inverso hexapeptide with hydrogen peroxide.

The desired peptide was then obtained by complete reduction using N-methylmercaptoacetamide (MMA) as described in R.A. Honghton and C.H. Li, Anal. Biochem. 98, 36 (1979). After treating the oxidised peptide with MMA for 50 hours at 37°C, a product is obtained, of which the chromatogram is characterised by a main peak corresponding to that of the reference standard.

16) Chromatographic purification and isolation of the pyroglutamylphenylalanyl-gem-diaminophenylalanylmalonylleucylmethionineamide.

The required product was isolated by reverse phase high pressure preparative liquid chromatography, the stationary phase being constituted by Lichroprep RP18 (25—40 µm) (Merck), and the eluent $H_2O$—$CH_3CN$ (80:20). The product, recovered by lyophilisation after evaporating $CH_3CN$, shows the same properties as the reference standard:

M.P. = 261—265°C

$[\alpha]_{22}^{546}$ = 10.0 (C = 0.5 in DMF)

Elementary analysis for $C_{36}H_{49}N_7O_7S$:

Theoretical: C, 59.75%; H, 6.77%; N, 13.55%;
Found: C, 59.67%; H, 6.69%; N, 13.49%.

Amino acid analysis:

Theoretical: Glp, 1.00; Phe, 1.00; Leu, 1.00; Met, 1.00;
Found: Glp, 1.04; Phe, 1.00; Leu, 1.00; Met, 0,93.

Chromatographic analysis (thin layer chromatography and HPLC) shows no presence of impurities, and the 1H n.m.r. spectrum confirms the molecular structure.

## Claims

1. A process for producing polypeptides containing one or more retro-inverted amide bonds, *entirely in the solid phase,* characterised by:

a) using the resin polydimethylacrylamide-co-acryloylsarcosine methylester, cross-linked with N,N′-ethylene-bis-acrylamide as the insoluble support for elongating the polypeptide chain in stages;

b) choosing a synthetic strategy which comprises assembling the polypeptide chains starting from the C-terminal residue by successive condensation of suitable derivatives of amino acids and/or peptides and of compounds corresponding to general formula:

(D)

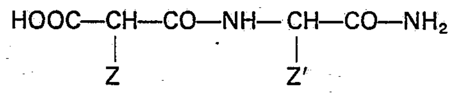

where Z and Z′, which can be the same or different, indicate the side-chain groups of the amino acids commonly present in natural polypeptide molecules;

c) using the reagent I,I-bis(trifluoroacetoxy)iodobenzene for the direct conversion of chain-terminal primary carboxyamide groups into amines;

d) finally simultaneously releasing the entire peptide from the resin, the terminal $NH_2$ protecting groups and the lateral functionalities compatible with the release conditions used.

2. The process as claimed in claim 1 for preparing the peptide:

$$Glp\text{-}Phe\text{-}gPhe\text{-}mGly\text{-}Leu\text{-}Met\text{-}NH_2$$

characterised by:

a) preparing the resin claimed under point 1, which by treatment with ethylenediamine is functionalised with primary amino groups at which the following have been condensed in succession:

i) norleucine residues as internal standards;

ii) the necessary "handle"

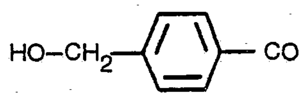

for condensing the C-terminal amino acid;

b) condensing at the hydroxyl of the "handle", methionine residues followed by leucine residues, with the amine functionalities blocked by $N^{\alpha}$-9-fluorenylmethoxycarbonyl groups;

c) preparing the intermediate of general formula I, where Z is H and Z′ is

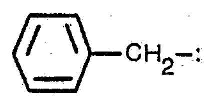

d) condensing the intermediate

(D)

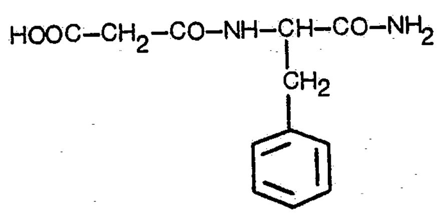

at the $NH_2^{\alpha}$— groups of the leucine residues;

e) treating the peptide bonded to the resin, with I,I-bis(trifluoroacetoxy)iodobenzene in a mixed aqueous-organic solvent;

f) condensing, in succession, phenylalanine residues and pyroglutamic acid at the free amino ends obtained in stage 2e; and finally

g) releasing the peptide chains from the resin by ammonolysis in methanol, followed by reduction of the methionine sulphoxide residues using N-methylmercaptoacetamide.

**Patentansprüche**

1. Zur Gänze in der Festphase ablaufendes Verfahren zur Herstellung von eine oder mehrere retroinvertierte Amidbindungen enthaltenden Polypeptiden, gekennzeichnet durch:

a) Verwendung des Harzes Polydimethylacrylamid-Co-acryloylsarcosinmethylester, vernetzt mit N,N′-Ethylen-bis-acrylamid, als unlöslicher Träger für die schrittweise Verlängerung der Polypeptidkette;

b) Auswahl einer Synthesestrategie, welche einen Aufbau der Polypeptidketten, ausgehend von dem C-endständigen Rest, durch aufeinanderfolgende Kondensation von geeigneten Derivaten von Aminosäuren und/oder Peptiden und von Verbindungen entsprechend der allgemeinen Formel:

(D)

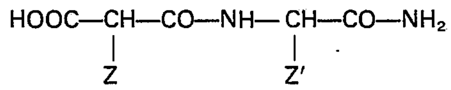

(I),

worin Z und Z', die gleich oder verschieden sein können, die Seitenkettengruppen der Aminosäuren angeben, die üblicherweise in natürlichen Polypeptidmolekülen vorliegen, umfaßt;

c) Verwendung des Reagenz J,J-Bis(trifluoracetoxy)iodobenzol für die direkte Umwandlung von kettenendständigen primären Carboxyamidgruppen in Amine;

d) schließlich gleichzeitiges Freisetzen des gesamten Peptids von dem Harz, wobei die Schutzgruppen für endständiges $NH_2$ und die seitenständigen Funktionalitäten mit den Freisetzungsbedingungen verträglich sind.

2. Verfahren nach Anspruch 1 zur Herstellung des Peptids:

Glp-Phe-gPhe-mGly-Leu-Met-$NH_2$,

gekennzeichnet durch:

a) Herstellung des in Anspruch 1 beanspruchtem Harzes, welches durch Behandlung mit Ethylendiamin mit primären Aminogruppen funktionalisiert wird, woran aufeinanderfolgend die folgenden Reste kondensiert worden sind:

i) Norleucinreste als interner Standard;

ii) der notwendige "Verbindungsstiel"

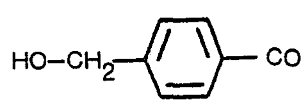

zum Kondensieren der C-endständigen Aminosäure;

b) Kondensieren, am Hydroxylende des "Verbindungsstiels", von Methioninresten und anschließend Leucinresten, wobei die Aminfunktionalitäten durch $N^{\alpha}$-9-Fluorenylmethoxycarbonylgruppen blockiert sind;

c) Herstellen des Zwischenproduktes der allgemeinen Formel I, worin Z Wasserstoff beduetet und Z' für

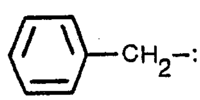

steht;

d) Kondensieren des Zwischenproduktes

(D)

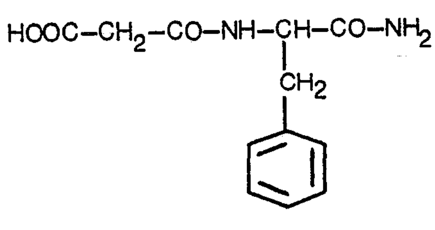

an die $NH_2^{\alpha}$-Gruppen der Leucinreste;

e) Behandeln des an das Harz gebundenen Peptids mit J,J-Bis(trifluoracetoxy)iodobenzol in einem gemischt wäßrig-organischen Lösungsmittel;

f) aufeinanderfolgendes Kondensieren von Phenylalaninresten und Pyroglutaminsäureresten an die in Stufe e) erhaltenen freien Aminenden; und schließlich;

g) Freisetzen der Peptidketten von dem Harz durch Ammonolyse in Methanol und anschließende Reduktion der Methioninsulfoxidreste unter Verwendung von N-Methylmercaptoacetamid.

**Revendications**

1. Procédé de synthèsise de polypeptides contenant une ou plusieurs liaisons amide retro-inversées *entièrement en phase solide,* caractérisé par le fait que:

a) on utilise une résine polydiméthylacrylamide-co-acryloylsarcosinate de méthyle, réticulée avec du N,N'-éthylène-bis-acrylamide, comme support insoluble pour l'allongement par étapes de la chaîne polypeptidique;

b) on choisit une voie de synthèse qui comporte l'assemblage des chaînes polypeptidiques à partir du reste C-terminal par condensations successives de dérivés d'acides aminés et/ou de peptides appropriés et de composés répondant à la formule générale:

(D)

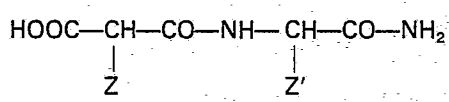

dans laquelle Z et Z', identiques ou différents, désignent les groupements latéraux des acides aminés courants dans les molécules de polypeptides naturels;

c) on utilise le réactif I,I-bis(trifluoroacetoxy)iodobenzène pour la conversion directe des chaînes terminales primaires carboxyamides en amines;

d) finalement, on libère simultanément de la résine, le peptide entier, les groupements de protection de $NH_2$ terminal et les fonctions latérales compatibles avec les conditions de libération employées.

2. Procédé selon la revendication 1 pour la préparation du peptide:

Glp-Phe-gPhe-mGly-Leu-Met-$NH_2$

caractérisé par le fait que:

a) on prépare la résine selon la revendication 1, qui, par traitement avec l'éthylènediamine, est fonctionnalisée avec des groupements amines primaires qui sont successivement condensés avec:

(i) des restes norleucine faisant office de standards internes;

(ii) le pont nécessaire

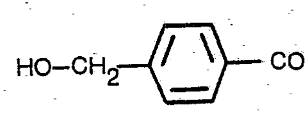

pour la condensation de l'acide aminé C-terminal;

b) on condense sur l'hydroxyle du pont, des restes methionine, puis des restes leucine, les fonctions amines étant bloquées par des groupements $N^{\alpha}$-9-fluorenylmethoxycarbonyles;

c) on prépare le composé intermédiaire de formule générale (I), dans laquelle Z désigne H et Z' désigne:

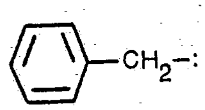

d) on condense le composé intermédiaire

(D)

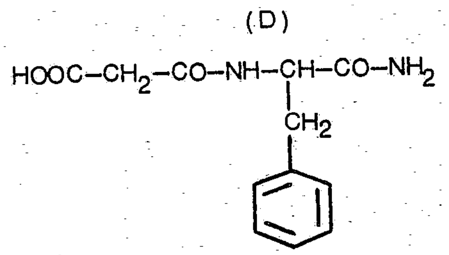

sur les groupements $NH_2^{\alpha}$ des restes leucine;

e) on traite le peptide lié à la résine avec du I,I-bis(trifluoroacetoxy)iodobenzène, dans un mélange solvant aqueux-organique;

f) on condense successivement les restes phenylalanine et acide glutamique aux extrémités amino libres obtenues lors de la deuxième étape; et finalement

g) on libère les chaînes de peptides de la résine par ammonolyse dans le méthanol, et on réduit ensuite les restes sulfoxide-methionine à l'aide de N-methylmercaptoacetamide.